Europäisches Patentamt

European Patent Office

Office européen des brevets

(11) Publication number: **0 224 287**
**A1**

(12) **EUROPEAN PATENT APPLICATION**

(21) Application number: 86201878.5

(22) Date of filing: 28.10.86

(51) Int. Cl.⁴: **C 12 N 15/00**
**A 01 H 1/00, C 12 N 5/00**
**C 12 N 1/20, C 12 P 21/02**
**//(C12N1/20, C12R1:41),**
**(C12N1/20, C12R1:01)**

(30) Priority: 29.10.85 NL 8502948

(43) Date of publication of application:
03.06.87 Bulletin 87/23

(84) Designated Contracting States:
AT BE CH DE FR GB IT LI LU NL SE

(71) Applicant: Rijksuniversiteit Leiden
Rapenburg 73
Leiden(NL)

(72) Inventor: Schilperoort, Robbert Adriaan
Antonie Duycklaan 10c
NL-2334 CD Leiden(NL)

(72) Inventor: Hooykaas, Paul Jan Jacob
Floris Versterlaan 12
NL-2343 RS Oegtgeest(NL)

(72) Inventor: Hoekema, Andreas
141 Sanfrancisco St. App. 3
CA 94005 Brisbane(US)

(72) Inventor: van Veen, Ronald Jacques Maria
Koestraat 15a
NL-2312 XH Leiden(NL)

(72) Inventor: den Dulk-Ras, Harmke
Gerard Brandstraat 24
NL-2332 AL Leiden(NL)

(74) Representative: van der Saag, Johannes et al,
OCTROOIBUREAU VRIESENDORP & GAADE P.O. Box 266
NL-2501 AW The Hague(NL)

(54) A process for the incorporation of foreign DNA into the genome of dicotyledonous plants.

(57) A process is disclosed for the incorporation of foreign DNA into the genome of dicotyledonous plants comprising infecting these plants or incubating dicotyledonous plant protoplasts with bacteria suitable or made suitable for that purpose, which are provided with one or more tumour-inducing plasmids or derivatives therefrom, originally originating from Agrobacterium, or from bacteria which contain the T-DNA originating from the above-meant plasmids, and/or the virulence genes originating from the above-mentioned plasmids, incorporated elsewhere in the bacterial DNA.

EP 0 224 287 A1

-1-

A process for the incorporation of foreign DNA into the genome of dicotyledonous plants.

The invention relates to a process for the incorporation of any DNA including genes into the genome of dicotyledonous plants in a artificial way. Herein use is made of a transfer system naturally present in the soil bacterium Agrobacterium tumefaciens and Agrobacterium rhizogenes. Present in these bacteria are respectively a tumour-inducing or Ti-plasmid and a root-including or Ri-plasmid. Via the transfer system a particular portion of the Ti- or Ri-plasmid is introduced into plant cells by the bacteria, whereupon this region which is called the T-region of the plasmid is incorporated stably into the plant genome. Starting from this natural process a two-component system is developed based upon portions of this type of plasmids essential for the transfer and integration of DNA. With this system which is called the "binary vector system", if present in bacteria suitable or made suitable for that purpose, any type of DNA can be incorporated into the genome of plant cells. The "binary vector system" consists of two components: 1) the virulence region of the Ti-plasmid which contains genes involved in the transfer of the T-region to the plant cell, 2) the intact wild-type T-region or an artificial T-region composed *in vitro*, in which a DNA at choice is positioned between what are called "border sequences". These border sequences are essential for the transfer and subsequent integration of the T-region in the plant genome. In the original system as described in Dutch patent application 83 00698 and European patent application 84 200239.6 both components are present in Agrobacterium strains as co-existent (compatible) units (plasmids) replicating separately from the chromosome. The use of such Agrobacteria for the genetic manipulation of monocotyledonous plants is described in Dutch patent application No. 83 01048 and European patent application 84200792.4. In an extension of the "binary vector system" it is laid down that it is not essential for its application that both components are present as plasmids in Agrobacterium (Dutch patent application 84 01780). Also when the T-region and/or the virulence region are present in the chromosome of Agrobacterium, such T-regions are transferred to the plant cell. In the present patent application the novel invention is described that not only Agrobacterium strains, provided with a binary vector system, are capable of transferring DNA to the plant. The system also functions if it is used in other bacterium species which naturally or artificially are provided with all those genes necessary for the interaction with the plant cell and the subsequent transfer of the T-region. This is shown with the aid of the use of the bacterium type Rhizobium.

The Ti-plasmid of Agrobacterium tumefaciens is responsible for the capacity of these bacteria of causing tumours on dicotyledonous plants. (Van Larebeke et al, Nature (London) 252, 169-170 (1974); Zaenen et al, J. Mol. Biol. 86, 109-127 (1974). The tumours develop because of the transfer of a specific portion of the Ti-plasmid (the T-region) to the plant cell, the integration of this T-region in the nuclear DNA of the cell and the expression of genes situated in this T-region (Chilton et al, Cell 11, 263-271 (1977); Willmitzer et al., Mol. Gen. Genet. 182, 255-261 (1981). The onc genes situated on the T-DNA code for enzymes involved in the biosynthesis of the plant hormones auxin and cytokinin (Schröder et al, Eur. J. Biochem. 138, 387-391 (1984), Akijoshi et al, Proc. Natl. Acad. Sci USA 81, 5994-5998 (1984), in consequence of which the transformed cell, in contrast to normal cells, can be grown on synthetic culture media without addition of these hormones (Braun et al, Proc. Natl. Acad. Sci USA 44, 344-349 (1958) and behave as tumour cells. In addition to the T-region that is found back in the plant cell a second region is essential for the virulence properties of the bacterium (Ooms et al, J. Bacteriol. 144, 82-91 (1980); Garfinkel et al, J. Bacteriol 144, 732-743 (1980)). Mutation of the virulence region provided seven vir-loci, called A, B, C, D, E, F and O (Klee et al, J. Bacteriol. 153, 878-883 (1983); Hille et al, Plasmid 7, 107-118 (1982); Hooykaas et al, Plasmid 11, 195-205 (1984). Mutations in these loci can all be complemented in trans by wild-type genetic information (Hille et al, Plasmid 7, 107-118 (1982); Hille et al, J. Bacterio. 158, 754-756 (1984); Klee et al, J. Bacteriol. 150, 327-331 (1982)). Some of the vir genes appear to code for excretory products (Otten et al, Mol. Gen. Genet., 195, 159-163 (1984)). In addition to plasmid genes there are also genes present

-4-

on the chromosome of Agrobacterium which are necessary for the virulence of the bacteria (Douglas et al, J. Bacteriol. 152, 1265-1275 (1982)).

It was realized at an early stage that this natural system of genetic manipulation of plants cells by Agrobacterium can be used to get foreign DNA along with the T-region into the plant, as a consequence of which plants having entirely new properties could be obtained (a.o. Schilperoort in Genetic Manipulations with plant material, Ed. L. Ledoux, Plenum Publishing Corporation, 141-162 (1975)). The following recent data on the molecular mechanism with which the natural process of tumour-induction proceeds have added to it that the Agrobacterium system now indeed is practically feasible for the genetic manipulation of plant cells and the regeneration of grown, sound plants having new properties.

1. DNA at choice which is inserted in the T-region is transferred along with the T-region to the plant cell and can be found back intact in the genome (Hernalsteens et al, Nature (London), 287, 654-656 (1980).

2. Removal of genes present in the T-region (among which the onc genes( have no effect on the transfer of the T-region, so that now in the absence of the onc-genes in the T-region complete, fertile plants can be regenerated from transformed plant cells (Leemans et al, EMBO. J. 1, 147-152 (1982); Hille et al, Plant mol. Biol., 2, 155-163 (1983)).

3. The virulence region and a T-region may be separated

0224287

-5-

physically from each other temporally or permanently withour the capacity of tumour induction or transfer of an artificial T-region being affected thereby. (Hoekema et al, Nature (London) 303, 179-180 (1983); de Framond et al, Biotechnology 1, 262-260 (1983); Dutch patent application 83 00698; European patent application 84200238.6).

4. Both the virulence region and the wild-type T-region or artificial T-region can be integrated separately or jointly into the chromosome of Agrobacterium without the capacity of transfer of such T-regions to the plant cell being affected (Hoekema et, EMBO J. 3, 2485-2490 (1984); Dutch patent application No. 84 01780, European patent application 85200871.3, U.S. patent application 737.154.

So far the systems for transfer of DNA at choice have be used with Agrobacterium as host. For various reasons, among which for instance extension of selection possibilities and/or host range, it is of practical use to be able to apply also other bacterial hosts for the transfer of DNA to the plant cell. Bacteria of the genus Rhizobium obtain after introduction of the Ti-plasmid the capacity of inducing tumours on dicotyledonous plants (Hooykaas et al, J. Gen. Microbiol. 98, 477-484 (1977)). They therefore contain just like Agrobacterium those chromosomal genes necessary for the virulence of the bacteria. In the present patent application experiments are described which show that systems derived from the Ti-plasmid for the introduction of foreign DNA into the plant cell of dicotyledonous plants (in particular the binary vector system) which are active in Agrobacterium can also be used in Rhizobium. Of the various possibilities earlier described by us for the use of a binary vector system the results are given here for a system, where T-region is

applied in the <u>Rhizobium</u> chromosome and the Vir-region
is present on a plasmid.

Experiments.

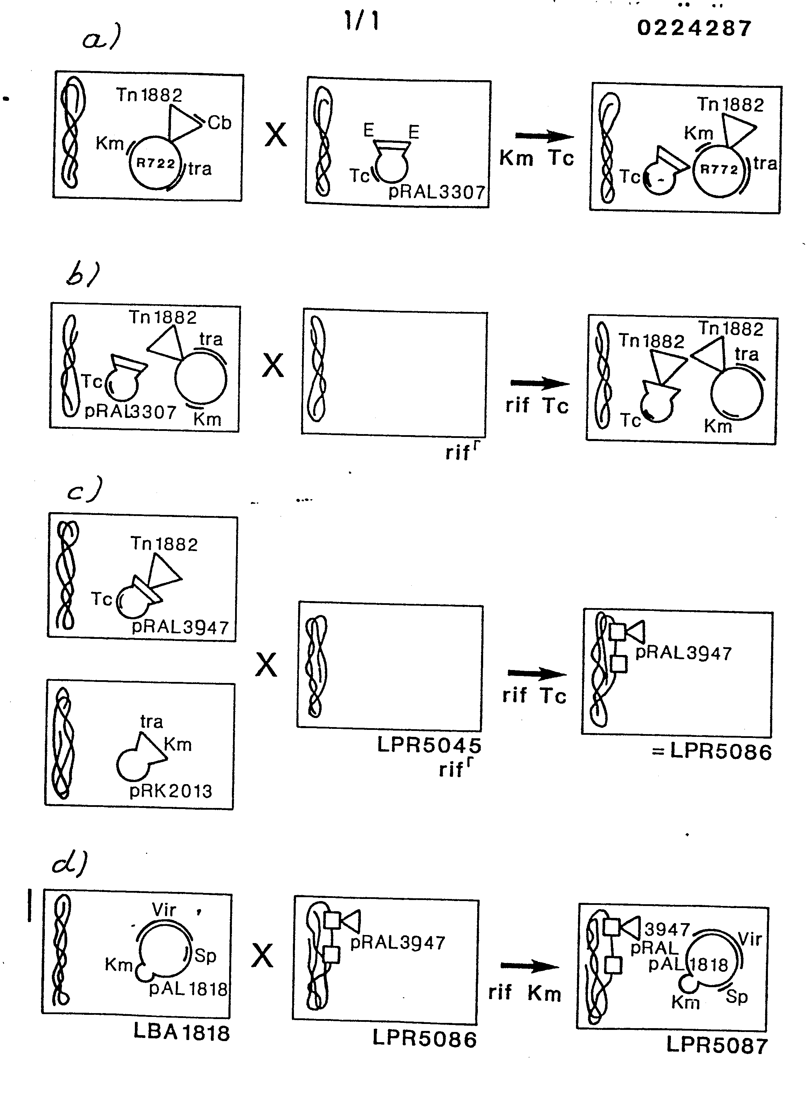

In order to show that the binary vector system also funct-
ions in <u>Rhizobium</u> the wild-type T-region of the Ti-plasmid
is in its intact form inserted in the chromosomal DNA
of <u>Rhizobium</u> trifolii (LPR5045). The approach followed
is represented in outline in fig. 1. As a first step by
means of what is called a shot gun cloning experiment
<u>Eco</u>RI restricted fragments originating from the chromosome
of LPR5045 were cloned in the <u>E.coli</u> vector pACYC184. Of
the clones obtained an isolate with an insertion of 5.4 kbp
chromosomal DNA was selected for further experiments. This
plasmid was called pRAL 3307. On this plasmid the T-region
of the Ti-plasmid was introduced by transposition of
Tn1882, a transposon derived from Tn3 in which the whole
T-region is closed, vide figure 1A, B. For the construction of Tn1882 reference is made to Hoekema et al, EMBO J.
3, 2485-2490 (1984). The plasmid pRAL3947 thus obtained
contained Tn1882 inserted in the <u>Rhizobium</u> chromosomal DNA
of pRAL3307. By means of an assisting plasmid pRK2013
(Figurski et al, Proc. Natl. Acad. Sci. USA, 76, 1648-
1652 (1979)) pRAL3947 was introducted into <u>Rhizobium</u>
<u>trifolii</u> (strain LPR5045) (fig. 1 C). Plasmid pRAL3947
cannot maintain itself in <u>Rhizobium</u> and gets lost unless
homologous recombination occurs via the 5.4 kbp <u>Eco</u>RI
fragment present on the vector and the chromosome of
LPR5045 where this fragment originates from. The resulting
<u>Rhizobium</u> <u>trifolii</u> strain is called LPR5086. DNA hybridisation studies showed that in this strain the T-region
is present intact and is maintained stably. This strain
does not yet contain the virulence genes of the Ti-plasmid and is therefore still avirulent. This virulence region was introduced by transferring the plasmid pAL1818, on which the vir genes A-E incl. and O are present (Hille et al. Plasmid 7, 107, 118 (1982)) via conjugation from LBA1818 to LPR5086. (Fig. 1 D). The resulting strain LPR5087 contains both components of the binary vector system and was tested for its cpacity of inducing tumours. LPR 5087 was tested on a number of plant species (such as, pea, tomato, tobacco) and turned out to be virulent on all these plants. Herewith it has been shown that the binary vector system described in Dutch patent applications 8300698 and 8401780) can also be used in Rhizobium bacteria. In addition we made the remarkable new invention that the transfer of a Ti-plasmid into non-oncogenic Phyllobacterium bacteria, resulting in strain LAZ100, made these bacteria oncogenic. Strain LAZ100 turned out to have the capacity to transfer and integrate the T-region of the Ti-plasmid into the genome of plant cells. LPR5087 and LAZ100 are deposited with the Centraal Bureau voor Schimmel cultures (CBS) at Baarn, Holland, under No. 768.85 on October 25, 1985 and on October 27, 1986 under No. (not yet known).

-1-

CLAIMS

1. A process for the incorporation of foreign DNA into the genome of plants, by infecting these plants or explants from them, or incubating the plant protoplasts or cells with bacteria suitable or made suitable for that purpose, characterized in that dicotyledonous plants are infected or dicotyledonous plant protoplasts are incubated with bacteria suitable or made suitable for that purpose, which are provided with one or more tumour-inducing plasmids or derivatives therefrom, originally originating from Agrobacterium, or from bacteria which contain the T-DNA originating from the above-meant plasmids, and/or the virulence genes originating from the above-mentioned plasmids, incorporated elsewhere in the bacterial DNA.

2. A process according to claim 1, characterized in that for the infection or incubation use is made of Rhizobium bacteria or Phyllobacterium bacteria.

3. A process according to claim 1 or 2, characterized in that bacteria are applied which are provided with one or more Ti- or Ri-plasmids or derivatives therefrom.

4. A process according to claim 3, characterized in that

-2-

the bacteria used have been provided with a stable cointegrate plasmid, constructed from a plasmid R772 and a plasmid pTiB6 with foreign DNA incorporated in the T-region of the latter.

5. A process according to any of the preceding claims, characterized in that bacteria are used, which contain at least one plasmid, which has the Vir-region of a tumour-inducing plasmid but no T-region, and at least one other plasmid, which has a T-region with incorporated therein foreign DNA but no Vir-region.

6. Dicotyledonous plants and plant cells obtained after, applying the process according to any of the preceding claims, the generic properties of the original plants or plant cells have been changed.

7. A process for the preparation of chemical and/or pharmaceutical products, characterized in that cells obtained with application of the process according to any of the claims 1-5 are cultivated and the desirable substance is isolated.

8. A process according to claim 7, characterized in that culturing is effected by means of fermentation and if useful subsequent immobilisation.

9. A process according to any of the claims 1-5 incl. or 8, characterized in that the regulator regions positions before and behind thd protein coding regions of T-DNA genes, in particular the genes for octopine synthesis for expressing foreign genes in dicotyledonous plant cells are used.

10. Dicotyledonous DNA having a portion artificially inserted in it with the process according to any of the preceding claims.

11. Cell lines and regenarated plants obtained after application of the process according to any of the claims 1-9.

12. _Rhizobium trifolii_ LPR 5087 and mutants thereof.

13. _Phyllobacterium_ LAZ100 and mutants thereof.

a)

Tn1882

Km — R722 — Cb — tra

X

E    E
Tc — pRAL3307

$\longrightarrow$ Km Tc

Tn1882

Km — R772 — tra
Tc

b)

Tn1882

tra

Tc — pRAL3307 — Km

X

rif$^r$

$\longrightarrow$ rif Tc

Tn1882    Tn1882

tra

Tc — Km

c)

Tn1882

Tc — pRAL3947

X

LPR5045
rif$^r$

$\longrightarrow$ rif Tc

pRAL3947

= LPR5086

tra
Km
pRK2013

d)

Vir

Km — pAL1818 — Sp

LBA1818

X

pRAL3947

LPR5086

$\longrightarrow$ rif Km

3947
pRAL
pAL 1818 — Vir

Km    Sp

LPR5087

European Patent Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 120 515 (RIJKSUNIVERSITEIT LEIDEN) <br><br> * Abstract; claims 1-6 * | 1,3,4 6-8,10 ,11 | C 12 N 15/00 <br> A 01 H 1/00 <br> C 12 N 5/00 <br> C 12 N 1/20 <br> C 12 P 21/02 // <br> (C 12 N 1/20 <br> C 12 R 1:41 ) <br> (C 12 N 1/20 <br> C 12 R 1:01 ) |
| Y | | 2,9,12 | |
| D,X | EP-A-0 120 516 (RIJKSUNIVERSITEIT LEIDEN) <br><br> * Abstract; page 12, lines 11-22; claims 1-7 * | 1,3,5 6,8,10 ,11 | |
| Y | | 2,9,12 | |
| D,X | EMBO JOURNAL, vol. 3, no. 11, 1984, pages 2485-2490, IRL Press Ltd, Oxford, GB; A. HOEKEMA et al.: "Delivery of T-DNA from the Agrobacterium tumefaciens chromosome into plant cells" <br> * Whole document * | 1,3,5 10 | **TECHNICAL FIELDS SEARCHED (Int Cl 4)** <br><br> C 12 N <br> C 12 P |
| Y | IDEM | 2 | |

--- -/-

The present search report has been drawn up for all claims

| Place of search | Date of completion of the search | Examiner |
|---|---|---|
| THE HAGUE | 25-02-1987 | MADDOX A.D. |

**European Patent Office**

**EUROPEAN SEARCH REPORT**

Application number

EP  86 20 1878

| | DOCUMENTS CONSIDERED TO BE RELEVANT | | Page  2 |
|---|---|---|---|
| Category | Citation of document with indication, where appropriate, of relevant passages | Relevant to claim | CLASSIFICATION OF THE APPLICATION (Int. Cl 4) |
| X | CHEMICAL ABSTRACTS, vol. 100, no. 25, June 1984, page 135, abstract no. 204333d, Columbus, Ohio, US; A. HOEKEMA et al.: "Transfer of the octopine T-DNA segment to plant cells mediated by different types of Agrobacterium tumor- or root-inducing plasmids: generality of virulence systems", & J. BACTERIOL. 1984, 158(1), 383-5 * Whole abstract * | 1,3,5, 10 | |
| Y | INTERNATIONAL REVIEW OF CYTOLOGY, supplement 13, 1981, pages 105-125, Academic Press Inc.; J.M. JAYNES et al.: "The position of Agrobacterium rhizogenes" * Page 122, paragraph 1 * | 2,12 | |
| X | PROCEEDINGS OF THE NATIONAL ACADEMY OF SCIENCES OF THE USA, vol. 80, no. 15, August 1983, pages 4803-4807, Washington, US; R.T. FRALEY et al.: "Expression of bacterial genes in plant cells" * Abstract; page 4803, right-hand column, paragraph 2 * | 1,3,6, 10 | |
| Y | IDEM | 9 | |
| | ---                    -/- | | |

The present search report has been drawn up for all claims

| Place of search | Date of completion of the search | Examiner |
|---|---|---|
| THE HAGUE | 25-02-1987 | MADDOX A.D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

**European Patent Office**

## DOCUMENTS CONSIDERED TO BE RELEVANT

| Category | Citation of document with indication. where appropriate, of relevant passages | Relevant to claim | CLASSIFICATION OF THE APPLICATION (Int. Cl.4) |
|---|---|---|---|
| Y | SCIENCE, vol. 222, 4th November 1983, pages 476-482; N. MURAI et al.: "Phaseolin gene from bean is expressed after transfer to sunflower via tumor-inducing plasmid vectors" <br> * Page 476, abstract; page 477, figure 1 * | 9 | |
| X | NUCLEIC ACIDS RESEARCH, vol. 12, no. 22, November 1984, pages 8711-8721, IRL Press Ltd, Cambridge, GB; M. BEVAN: "Binary Agrobacterium vectors for plant transformation" <br> * Abstract; page 8719, last paragraph - page 8720, paragraph 1 * | 1,3,5, 6,10 | |
| A | IDEM | 11 | TECHNICAL FIELDS SEARCHED (Int Cl 4) |
| D,P X | EP-A-0 159 418 (RIJKSUNIVERSITEIT LEIDEN) <br> * Claims 1-9 * | 1-12 | |
| P,X | EP-A-0 176 112 (RIJKSUNIVERSITEIT LEIDEN) <br><br> * Claims 1-5 * | 1,3,5, 6,10, 11 | |

-/-

The present search report has been drawn up for all claims

| Place of search | Date of completion of the search | Examiner |
|---|---|---|
| THE HAGUE | 25-02-1987 | MADDOX A.D. |

0224287

European Patent
Office

EUROPEAN SEARCH REPORT

Application number

EP  86 20 1878

## DOCUMENTS CONSIDERED TO BE RELEVANT

Page  4

| Category | Citation of document with indication, where appropriate, of relevant passages | Relevant to claim | CLASSIFICATION OF THE APPLICATION (Int. Cl.4) |
|---|---|---|---|
| A | BIOLOGICAL ABSTRACTS, vol.. 56, 1972, page 5332, abstract no. 52745, Biological Abstracts Inc., Philadelphia, US; H.T. HORNER et al.: "Nomenclature of bacteria in leaf nodules of the families Myrsinaceae and Rubiaceae", & INT. J. SYST. BACTERIOL 22(2): 117-122, 1972 | | |
| A | BIOLOGICAL ABSTRACTS, vol. 64, 1977, page 5346, abstract no. 54583, Biological Abstracts Inc., Philadelphia, US; J. DE SMEDT et al.: "Intra- and intergeneric similarities of Agrobacterium ribosomal ribonucleic acid cistrons", & INT. J. SYST. BACTERIOL 27(3): 222-240, 1977 | | TECHNICAL FIELDS SEARCHED (Int. Cl.4) |

The present search report has been drawn up for all claims

| Place of search | Date of completion of the search | Examiner |
|---|---|---|
| THE HAGUE | 25-02-1987 | MADDOX A ). |